# EUROPEAN PATENT APPLICATION

(11) **EP 4 428 867 A1**
(43) Date of publication of application: **11.09.2024**
(21) Application number: 23160786.2
(22) Date of filing: 08.03.2023
(51) Int. Cl.: G16H 10/60

(54) **SYSTEM AND METHOD FOR MERGING MULTIPLE DE-IDENTIFIED MEDICAL DATASETS**

(71) Applicant: Koninklijke Philips N.V., 5656 AG Eindhoven (NL)
(72) Inventor: JAIN, Anshul, Eindhoven (NL); GOOSSENS, Joël, Eindhoven (NL); BUCUR, Anca Ioana Daniela, 5656AG Eindhoven (NL); PAWAR, Shreeyash Sanjay, Eindhoven (NL)
(74) Representative: Philips Intellectual Property & Standards

(57) **Abstract**

Some embodiments are directed to merging multiple de-identified medical datasets. A knowledge graph representing information in the multiple datasets may be constructed and an entity resolution algorithm executed. A further de-identification may be performed for patient nodes that have a re-identification risk exceeding a re-identification risk threshold.

## Description

### TECHNICAL FIELD

The presently disclosed subject matter relates to a method for merging multiple de-identified medical datasets, a system for merging multiple de-identified medical datasets, a computer readable medium.

### BACKGROUND

Privacy legislations, such as the Health Insurance Portability and Accountability Act of 1996 (HIPAA), generally include rules that set strict guidelines with respect to de-identification of Protected Health Information (PHI) from the medical data to be shared. The data risk is the probability of re-identification risk in the scenario where the data is exposed to an attack.

For example, the HIPAA Privacy Rule states that only individually identifiable information is covered by the regulation. It goes on to state that data is no longer subject to the regulation when it has been de-identified. Specifically, this is defined as, "Health information that does not identify an individual and with respect to which there is no reasonable basis to believe that the information can be used to identify an individual is not individually identifiable health information."

In this exemplary context, reference is made to:
Direct identifiers: attributes that uniquely identify the individual.
Quasi-identifiers: attributes that, in combination, can be linked with external information to re-identify, or reduce uncertainty about, all or some of the respondents to whom information refers,
Sensitive data: Sensitive data is the classified information that should be protected and is inaccessible to outside parties unless specifically granted permission.

The simplest way of de-identification includes the process of removing direct identifiers from a dataset to reduce the ability to identify individuals (e.g., HIPAA 18 identifiers). Direct identifiers are fields that can be used alone to uniquely identify individuals or their households, e.g., medical record number, phone number. De-identification may also include treating other identifiers in the dataset like the quasi identifiers and sensitive attributes.

A growing collection of investigations demonstrate how de-identified information can be re-identified to the individuals from which it was derived, e.g., via demographics, genome sequences, mobility patterns, and social networks, graph generation, etc.

### SUMMARY

Knowledge graphs are a way of representing information that can capture complex relationships more easily than conventional databases. A knowledge graph may be constructed from different heterogeneous data sources. Given the potential wealth of insights in personal data the big databases can provide, many organizations aim to share data while protecting privacy by sharing de-identified data but are concerned because various demonstrations show such data can be re-identified.

The inventors found that medical datasets may be represented as a knowledge graph. In particular, multiple medical datasets may be included in a single knowledge graph. This advantageous as the combined graph has a richer representation and as such may be more useful for research and other purposes. On the other hand, knowledge graphs may be used to make inferences about the nodes in the graph. The inventors found that nodes representing patients that were imported into the graph from different data set may sometimes nevertheless be identified as corresponding to the same person. For example, the two patient nodes may each be connected to attribute nodes that make it likely that the two patient nodes in fact correspond to the same person. However, once this identification has been performed the rest of the information associated with the nodes may also be pooled. As a result, now much more information is available about this person than previously. This may mean that the person can now be re-identified, or at least that the risk upon this happening is now increased.

An embodiment provides an approach to calculate re-identification risk after multiple knowledge graphs are merged and entity resolution is performed. Entities linked by the entity resolution may be used as identifier for re-identification risk determination. This information may be used to generate a warning and/or to further de-identify the data.

An embodiment of the method may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

Another aspect of the presently disclosed subject matter is a method of making the computer program available for downloading.

### BRIEF DESCRIPTION OF DRAWINGS

Further details, aspects, and embodiments will be described, by way of example only, with reference to the drawings. Elements in the Figs are illustrated for simplicity and clarity and have not necessarily been drawn to scale. In the Figs, elements which correspond to elements already described may have the same reference numerals. In the drawings,
Fig. 1 schematically shows an example of an embodiment of a system for merging multiple de-identified medical datasets,
Fig. 2a schematically shows an example of an embodiment of constructing a knowledge graph,
Fig. 2b schematically shows an example of an embodiment of a knowledge graph,
Fig. 2c schematically shows an example of an embodiment of constructing a knowledge graph,
Fig. 2d schematically shows an example of an embodiment of a knowledge graph,
Fig. 2e schematically shows an example of an embodiment of a knowledge graph,
Fig. 2f schematically shows an example of an embodiment of a knowledge graph,
Fig. 2g schematically shows an example of an embodiment of a knowledge graph,
Fig. 2h schematically shows an example of an embodiment of a knowledge graph,
Fig. 3a schematically shows an example of an embodiment of a knowledge graph,
Fig. 3b schematically shows an example of an embodiment of a knowledge graph,
Fig. 3c schematically shows an example of an embodiment of a knowledge graph,
Fig. 3d schematically shows an example of an embodiment of a knowledge graph,
Fig. 3e schematically shows a detail of an example of an embodiment of a knowledge graph,
Fig. 4 schematically shows an example of an embodiment of a method for merging multiple de-identified medical datasets,
Fig. 5a schematically shows a computer readable medium having a writable part comprising a computer program according to an embodiment,
Fig. 5b schematically shows a representation of a processor system according to an embodiment.

### Reference signs list

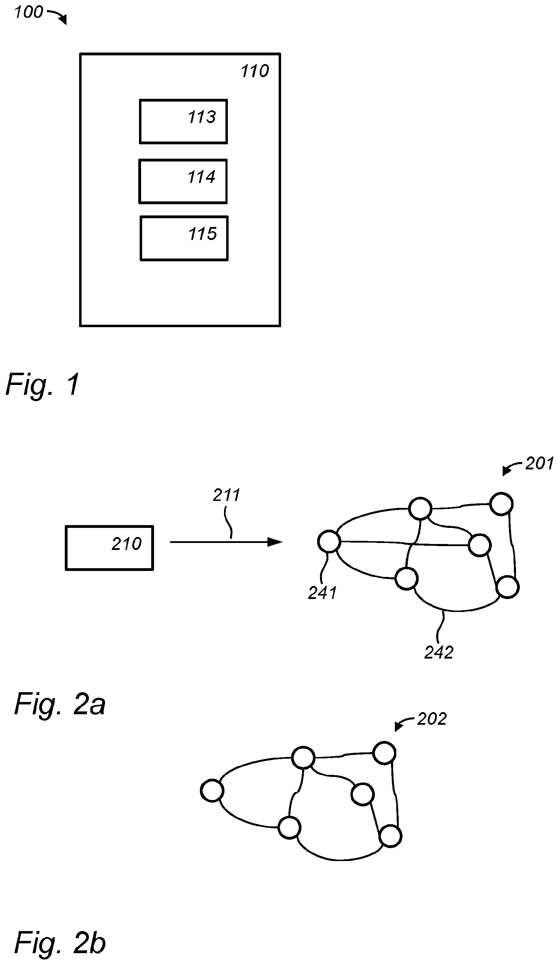

The following list of references and abbreviations corresponds to Figs 1-3e, 5a-5b, and is provided for facilitating the interpretation of the drawings and shall not be construed as limiting the claims.
- 100: a system
- 110: a merging device
- 113: a processor system
- 114: storage
- 115: communication interface
- 210, 212: a medical dataset source
- 211, 213: constructing a knowledge graph
- 201-206: a knowledge graph
- 221: associated nodes
- 231: a modified edge
- 241: a node
- 242: an edge
- A, B: matched nodes
- 301-303: knowledge graph
- 310: common attribution nodes
- 331-334: attribution nodes
- 321-324: patient nodes
- 311-313: common attribution nodes
- 341-344: patient nodes
- 351-353: attribution nodes

- 1000: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described.

In the following, for the sake of understanding, elements of embodiments are described in operation. However, it will be apparent that the respective elements are arranged to perform the functions being described as performed by them.

Further, the subject matter that is presently disclosed is not limited to the embodiments only, but also includes every other combination of features described herein or recited in mutually different dependent claims.

Fig. 1 schematically shows an example of an embodiment of a merging device 110. Merging device 110 is configured for merging multiple de-identified medical datasets. Merging device 110 may be part of a system 100. For example, system 100 may comprise further devices, e.g., medical dataset sources, e.g., database servers, e.g., client devices for consulting or visualizing a knowledge graph created by merging device 110.

A knowledge graph, also known as a semantic network, represents a network of nodes joined by edges. The nodes may represent real-world entities, e.g., they may correspond to objects, events, situations, or concepts. The edges illustrate the relationship between the nodes. A knowledge graph is stored in a data structure arranged for knowledge graphs, usually in a graph database. A knowledge graph may be visualized as a graph structure.

Merging device 110 may be configured to merge multiple de-identified medical datasets and to construct a knowledge graph therefrom. However, as the inventors found, such a knowledge graph may have a larger re-identification risk than the individual medical datasets, even if they were de-identified. Merging device 110 may be configured to identify this increased risk and/or to modify the constructed knowledge graph to alleviate this risk.

For example, in an embodiment the device 110 or system 100 may be used for medical research. For example, Medical knowledge graphs, especially knowledge graphs comprising large amounts of data may be used to improve health care. For example, knowledge graphs enable better insight in the health care quality and efficiency. These insights may then be used to improve care. Knowledge graphs are also useful to compare the effectiveness of different interventions, or monitor drug and device safety. In an embodiment, the device is configured to compare patients with one another using the knowledge graph. For example, a medical practitioner may use a knowledge graph to find previous patients that resemble a current patient. The knowledge graph may be used to compare the treatments the previous patients received, e.g., how effective it was. In an embodiment, the device is further arranged to train artificial intelligence tools using the knowledge graph, e.g., to find patterns in the data, e.g., to predict future development for a given patient, etc.

Merging device 110 may comprise a processor system 113, a storage 114, and a communication interface 115. Storage 114 may be, e.g., electronic storage, magnetic storage, etc. The storage may comprise local storage, e.g., a local hard drive or electronic memory. Storage 114 may comprise non-local storage, e.g., cloud storage. In the latter case, storage 114 may comprise a storage interface to the non-local storage. Storage may comprise multiple discrete sub-storages together making up storage 114. Storage may comprise a volatile writable part, say a RAM, a non-volatile writable part, e.g., Flash, a non-volatile non-writable part, e.g., ROM.

In the various embodiments of communication interfaces 115, the communication interfaces may be selected from various alternatives. For example, the interface may be a network interface to a local or wide area network, e.g., the Internet, a storage interface to an internal or external data storage, an application interface (API), etc.

Storage 114 may be non-transitory storage. For example, storage 114 may store data in the presence of power such as a volatile memory device, e.g., a Random Access Memory (RAM). For example, storage 114 may store data in the presence of power as well as outside the presence of power such as a non-volatile memory device, e.g., Flash memory.

The device 110 may communicate internally, with other systems, with other devices, external storage, input devices, output devices, and/or one or more sensors over a computer network. The computer network may be an internet, an intranet, a LAN, a WLAN, etc. The computer network may be the Internet. The device 110 comprise a connection interface which is arranged to communicate within system 100 or outside of system 100 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna.

The communication interface 150 may be used to send or receive digital data, e.g., to receive a medical data set, to send a knowledge graph, to receive information on knowledge graph, e.g., quasi identifiers that need to be taken into account for risk assessment and/or de-identification, etc.

Merging device 110 may have a user interface, which may include well-known elements such as one or more buttons, a keyboard, display, touch screen, etc. The user interface may be arranged for accommodating user interaction for performing a merge of medical data sets, displaying results, inputting risk thresholds, quasi identifiers, and the like.

The execution of device 110 may be implemented in a processor system. The device 110 may comprise functional units to implement aspects of embodiments. The functional units may be part of the processor system. For example, functional units shown herein may be wholly or partially implemented in computer instructions that are stored in a storage of the device and executable by the processor system.

The processor system may comprise one or more processor circuits, e.g., microprocessors, CPUs, GPUs, etc. Device 110 may comprise multiple processors. A processor circuit may be implemented in a distributed fashion, e.g., as multiple sub-processor circuits. For example, device 110 may use cloud computing.

Typically, the merging device 110 comprises a microprocessor which executes appropriate software stored at the device; for example, that software may have been downloaded and/or stored in a corresponding memory, e.g., a volatile memory such as RAM or a non-volatile memory such as Flash.

Instead of using software to implement a function, device 110 may, in whole or in part, be implemented in programmable logic, e.g., as field-programmable gate array (FPGA). The device may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, merging device 110 may comprise circuits, e.g., for cryptographic processing, and/or arithmetic processing. In hybrid embodiments, functional units are implemented partially in hardware, e.g., as coprocessors, and partially in software stored and executed on the device.

Expanding a knowledge graph often involves integrating it with another knowledge graph and that brings the concern of re-identification risk due to linking of multiple datasets that correspond to those knowledge graphs, which may contain sensitive attributes. The knowledge graphs can provide an early indication whether combining the corresponding de-identified datasets may increase the reidentification risk.

As soon as multiple knowledge graphs are linked a re-identification risk may be calculated, using the linked entities as identifier for re-id risk determination, possibly based upon the inputs from a de-identification expert. This information may be used to alert the system to take actions for the data e.g., further de-identification and/or generating a warning.

Using a knowledge graph, new relationships between nodes may be inferred using the data available in the knowledge graph. The new relationships that are found may in turn be used to find yet more relationships between nodes. In particular, two distinct nodes in the knowledge graph may be inferred to correspond to the same entity. Likewise, labels or information associated with a first node or a first edge may be inferred to hold for a second node or a second node as well. As a result, nodes may be linked, and information and/or label may be propagated.

Such inferences can reveal additional properties on the existing patient base and should therefore be properly assessed to prevent potential privacy breaches. Identifying such re-identification risks is difficult if the knowledge graph is built from de-identified data.

For example, after constructing a knowledge graph from two or more de-identified dataset, a first patient node and a second patient node may be identified in the knowledge graph, the first patients node and a second patient node both being connected to the same set of common attribute nodes, the first patient node being connected to a first set of attribute nodes to which the second patient node is not connected, the second patient node being connected to a second set of attribute nodes to which the first patient node is not connected. Based on the set of common attributes nodes to which the first and second node are connected, the merging device may conclude that the first patient node and the second patient node correspond to the same anonymized patient. The first patient node and the second patient node may then be linked. The new linked node is connected to the set of common attribute nodes, the first set of attribute nodes and the second set of attribute nodes. It may be that the re-identification risk is sufficiently small based only on the common attribute nodes and the first set of attribute nodes or based only on the common attribute nodes and the second set of attribute nodes, but that the re-identification risk for the linked patient node is now too high based on the common attribute nodes, the first set of attribute nodes, and the second set of attribute nodes. Appropriate action may then be taken, e.g., generate warning, perform further de-identification.

Consider for comparison, a tabular dataset built from multiple datasets that have not been de-identified but contain identifying information. In this case, the merging of databases results in collated datasets based on common patient identifiers. The collated data may then be de-identified using conventional means. In knowledge graph, the merging it not simple index based, and requires linking of many entities that forms relationships. Hence, re-identification risks are not as apparent for merged knowledge graphs.

Fig. 2a schematically shows an example of an embodiment of constructing a knowledge graph 201. Shown is a medical dataset source 210. From the dataset a knowledge graph 201 is constructed 211.

For example, source 210 may be a server from which the dataset may be downloaded. For example, source 210 may be storage medium from which the dataset may be retrieved. In this embodiment, it is assumed that the knowledge graph is constructed iteratively by expanding the knowledge graph with additional datasets. Alternatively, the knowledge graph could be made directly from a set of multiple datasets.

Before publishing a dataset or/and using it in HealthCare research, a data de-identification process is typically provided to ensure that no sensitive information about specific individuals is disclosed. This is achieved by substituting the original dataset by a modified dataset. There can be different sources of data including DICOM, EMR, FHIR, etc. De-identification may involve the removal or modification of identifying information. For example, the dataset may be de-identified following HIPAA guidelines for deidentification.

The datasets may be structured or unstructured. In this embodiment, the focus is on structured datasets, though unstructured datasets are also possible. For example, heterogeneous patient data may be pre-processed and assembled into a structured dataset. The structured dataset may be modelled as a table where each row corresponds to a patient and each column contains information about one of multiple attributes.

The knowledge graph may represent information in the datasets in various ways. A knowledge graph has nodes and edges. Part of the nodes represent anonymized patients; these are referred to as patient nodes. Part of the nodes represent patient attributes. Edges represent relationships between the nodes. Nodes and edges may be labelled or tagged with additional information. In Fig. 2a, one node is indicated with reference numeral 241 and one edge is indicated with reference 242. There may be at 1000, at least 10000, or more, patient nodes and/or attribute nodes in a knowledge graph.

In addition to the data in the dataset, metadata of the medical dataset may also be stored. For example, the metadata may indicate direct identifiers and quasi-identifiers. Information about identifiers in the dataset that are considered to be directly identifying or that are quasi identifying may later be used when computing re-identification risk. For example, the information may be stored in a separate table associated with the knowledge graph. The information may be stored in the knowledge graph itself. For example, in an embodiment, an attribute node may be a labelled as quasi identifying; nodes could be labelled as identifying as well, but it is assumed that such information would have been removed from the data set before it is obtained, or that such information would be removed during or after the construction of the knowledge graph.

Metadata may be stored as part of a knowledge graph ontology that contains information about the original dataset, quasi-identifiers and the risk threshold for individual datasets. This metadata information can later be referred to confirm that the datasets adhere to a certain level of privacy, which may or may not be sufficient for a certain use case.

Nodes and edges in a knowledge graph may be tagged with labels, representing additional information, e.g., their different roles in the dataset, e.g., that the node represents a patient, a disease, or other attribute. Nodes may store any number of key-value pairs, or properties. For example, a node representing high-blood pressure, may have an additional property with the value of the blood pressure. The edges in the knowledge graph may represent relationships; they may be directed or undirected. An edge may also be labeled with a label, e.g., indicating further information about the relationship. Relationships may be stored with, e.g., a type, a start node, and an end node, and properties, if any.

The knowledge graph may be represented in a storage, e.g., an electronic memory, in a data structure arranged for a knowledge graph. For example, the knowledge graph may be stored in a graph database. A graph database stores nodes and relationships instead of tables, or documents.

For example, the data structure may store a list of nodes, and for each node, an adjacency list or adjacency set. In an adjacency list representation of a graph, every node is represented as a node object. The node may contain data and/or a reference to a linked list. This linked list provides a list of all nodes that are adjacent to the current node, e.g., connected with an edge. The adjacency set is similar to adjacency list except for the difference that instead of a linked list; a set of adjacent vertices is provided.

Fig. 2b schematically shows an example of an embodiment of a knowledge graph.

Once a knowledge graph has been constructed from the dataset, the dataset could be discarded. Typically, the dataset is already de-identified when it is obtained, but the desired level of de-identification may be different. As a result at this point an optional de-identification may be performed. For example, in Fig. 2b, the original data set has been discarded, although it could still be available at the source 210. Furthermore, an optional additional de-identification has been done, shown in Fig. 2b by removing an edge from the knowledge graph. The modified knowledge graph thus represents less information compared to the knowledge graph of Fig 2a. Instead of removing an edge, the properties of the edge could be modified. Possibly, even edges could be added to de-identify other nodes.

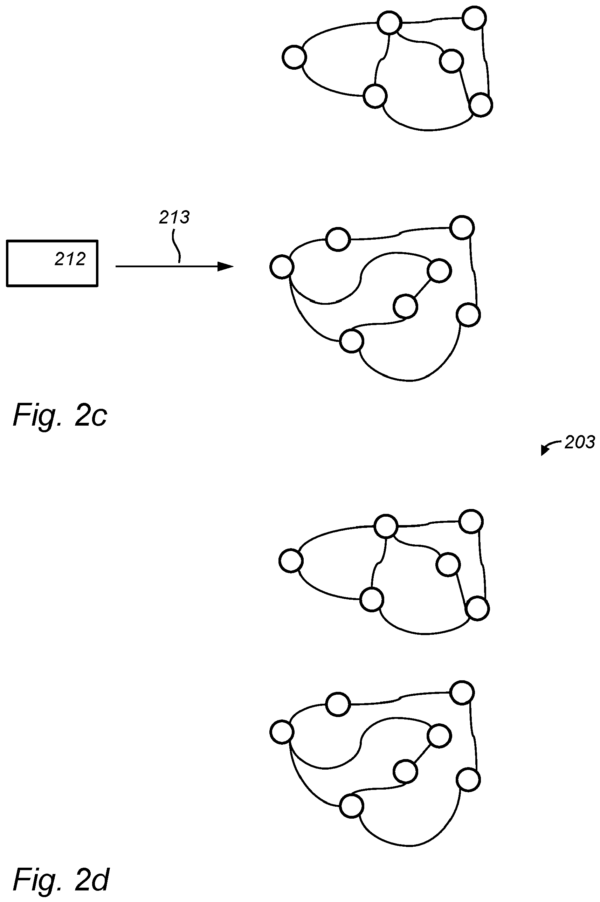

Fig. 2c schematically shows an example of an embodiment of constructing a knowledge graph. Shown is a further medical dataset source 212. From source 212 another medical dataset is obtained. Like Fig. 2a, the new dataset is also typically de-identified. However, the de-identified dataset could be de-identified as part of the obtaining. In this way, the knowledge graph is built multiple phases, as each dataset is added, the knowledge graph is expanded. This is shown in Fig. 2c, as the knowledge graph in Fig. 2b is present, but a new cluster is now added. As the knowledge graphs are de-identified, the constructed knowledge graph may be relatively unconnected, as many nodes that could be used for joining the graphs together have been removed due to the de-identifying; for example, direct identifiers have been removed or altered. However, as the information in the new datasets is inserted into the knowledge graph some nodes may be common. For example, both datasets may be structured in the same way, e.g., following a common ontology at least in part. For example, both knowledge graphs may have a node indicating strokes. If such nodes are common to both datasets and/or knowledge graphs, then no new nodes for stroke needs to be created when the new information is inserted. In Fig. 2c, no such common nodes are shown, but they may be used.

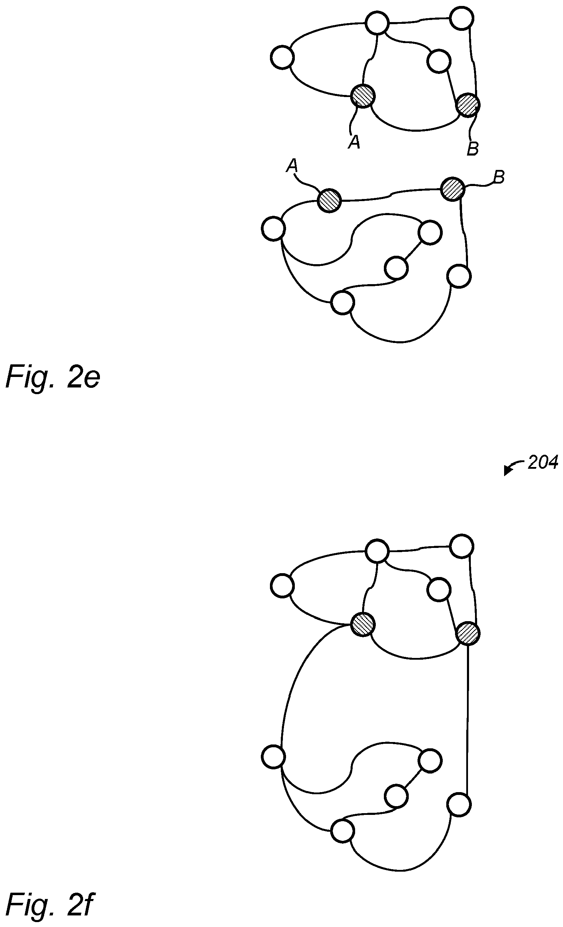

Fig. 2d schematically shows an example of an embodiment of a knowledge graph 203. Knowledge graph 203 is obtained from Fig. 2c, e.g., by discarding the datasets, and/or by further deidentification operations.

Fig. 2e schematically shows an example of an embodiment of a knowledge graph. The knowledge graph may be generated from de-identified datasets. This may be done iteratively, as shown in the Figs, or this may be done by first collecting all datasets, and constructing the knowledge graph from all datasets together. There are numerous ways in which a knowledge graph can be constructed from a technical standpoint. For example, a de-identified dataset may be mapped to a harmonized semantic data model, in case this has not been done prior.

Following the expanding of the knowledge graph an entity resolution algorithm may be executed to determine a set of nodes that each represent the same anonymized patient. For example, two nodes may be identified in the knowledge graph that each represents patients. It may be determined that the two nodes appear to represent the same patient with a likelihood exceeding a threshold.

In Fig. 2e, two such sets have been identified. The first set comprises two nodes, each labeled 'A'. The second set comprises two nodes, each labeled 'B'. The entity resolution may work in various ways. There may be sets with more than two nodes, that each seem to correspond to the same anonymized patient.

For example, the entity resolution algorithm may apply a similarity function to a pair of nodes. If the similarity function indicates a high likelihood they may be linked or matched. Below various ways of linking nodes are indicated.

Multiple similarity functions may be used. For example, a first similarity function may determine easier cases, while a second similarity function may do harder cases. For example, different similarity functions may use different algorithms, e.g., for different cases. For example, the first similarity function may find nodes that indicate the same attribute. Different algorithms may be used for this. For example, the first similarity function may identify attribute nodes with the same name label, e.g., cancer. For example, the first similarity function may map onto each other two ontologies that were used for the respective knowledge graphs. Again, a result of this may be that nodes are linked that each indicate the same disease, e.g., stroke. The second similarity function may identify two or more patient nodes that correspond to the same person, e.g., the same anonymized person. Multiple patient nodes may correspond to the same real person, e.g., the same un-anonymized person. The latter may be the result of merging multiple de-identified datasets into the same knowledge graph.

A similarity function, e.g., the second similarity function, may identify nodes that are connected to many of the same nodes. In an embodiment, a similarity function comprises a machine learning model trained to identify nodes that indicate the same person. For example, such a model could be trained on data for which the true identification of nodes is known.

In an embodiment, an initial re-identification risk for anonymized patients in the knowledge graph may be determined before an entity resolution algorithm. The entity resolution algorithm may then be performed for those anonymized patients that have an initial re-identification risk exceeding a threshold. This makes the entity resolution more efficient, as fewer nodes need to be considered. Although this may have some impact on the eventual re-identification risk that is computed, the impact is small. Especially those nodes for which information is available, so that de-identification is almost possible, are also the nodes most likely to exceed a risk threshold if any further information were added to these nodes as a result of executing entity resolution.

Note that there may be multiple entity resolution algorithms performed, e.g., using different similarity functions.

For example, in an embodiment, a first entity resolution algorithm is performed, e.g., using the first similarity function. This algorithm works on attribute nodes. A re-identification risk is then computed to identify high risk patient nodes; that is patient nodes for which re-identification is higher than a threshold. A second entity resolution algorithm is then performed, e.g., using a second similarity function, on the high risk patient nodes. For example, for each high risk patient node, a search is made in the full knowledge graph for a patient node that appears to correspond to the same patient.

Fig. 2f schematically shows an example of an embodiment of a knowledge graph 204. After nodes are identified as the same entity, e.g., the same patient or the same attribute, they are linked in knowledge graph. There are various ways to do this. For example, in knowledge graph 204 the identified nodes are joined. Edges between nodes that are joined may be joined as well. The latter is not necessary, but if this is not done there may be multiple edges joining two nodes.

An advantage of joining nodes and/or edges is that the knowledge graph size is reduced so that resulting algorithms runs faster, e.g., an additional entity resolution algorithm, or re-identification risk algorithm.

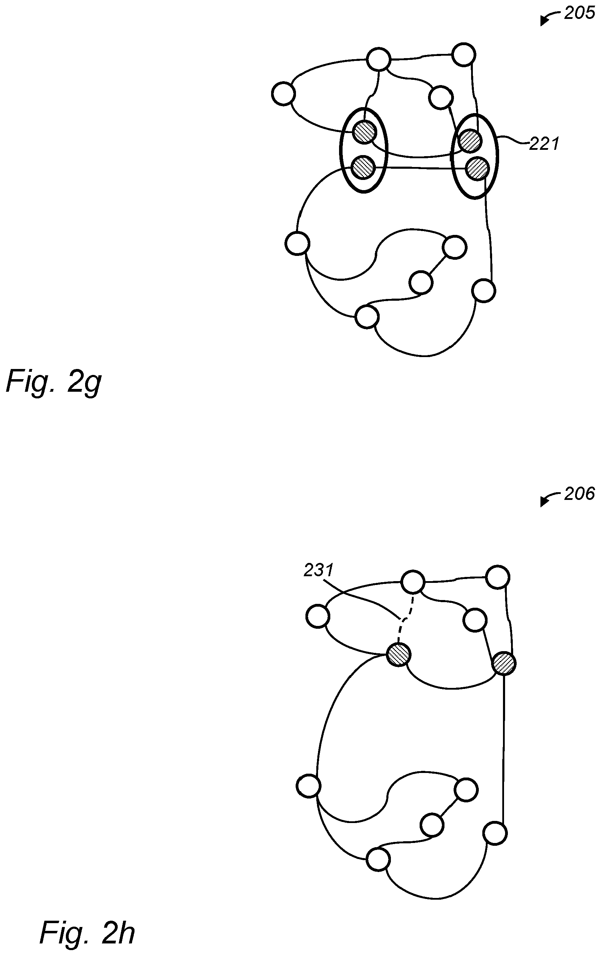

Fig. 2g schematically shows an example of an embodiment of a knowledge graph 205. In knowledge graph 205 nodes that were initially identified as corresponding to the same entity, e.g., the A-set and the B-set are not joined, but are associated with an additional data structure that indicate the relationship. Fig. 2g shows a relationship 221 that indicates that the nodes labeled B in Fig. 2e are the same. These relationships may be stored in a table, or may be incorporated in the knowledge graph. For example, an additional edge may be added between two similar nodes that indicates that the two nodes are presumably the same. For example, a property of the edge may indicate sameness. An advantage of this approach is that the edge can also express a likelihood. The sameness likelihood may be used in calculating the re-identification risk. For example, linked nodes with a lower sameness likelihood may receive a smaller re-identification risk, as there is additional uncertainty about the link between the nodes.

In an embodiment, an association between nodes, e.g., a sameness edge, is only added if the likelihood that the two nodes represent the same entity exceeds a threshold. The approach to similar nodes need to be the same for each iteration of the entity resolution, e.g., for each similarity function. For example, attributes nodes may be joined, while patient nodes may be associated without joining. For example, nodes may be joined if the likelihood of sameness exceeds a first threshold, while they may be associated if the likelihood exceeds a second threshold, but not the first threshold.

Fig. 2h schematically shows an example of an embodiment of a knowledge graph 206. Fig. 2h continues from Fig. 2f, e.g., having joined nodes, though the same could be done for Fig. 2g, e.g., having associated nodes. In an embodiment, a hybrid knowledge graph is used, using both joined and associated nodes.

After running the one or more entity resolution algorithms a re-identification risk for patient nodes may be computed. For example, the re-identification risk may be computed for those nodes that were linked by the entity resolution algorithm. Linked nodes likely have more information, e.g., more edges, than before so that the re-identification risk is likely increased.

The re-identification risk may also be computed for nodes that did not change. Consider two patient nodes: node 1 and node 2. Node 2 was linked to another node. Additional information is now available for node 2. As a result node 2 may have an increased re-identification risk. Node 1 was not linked, but may still have an increased re-identification risk. The reason is that previously node 2 may have been quite similar to node 1. This may reduce re-identification risk for node 1: the information available may not be sufficient to distinguish between the real persons corresponding to these nodes. But as node 2 now has more information, it may now be distinguishable from node 1. In an embodiment, only patient nodes that were linked are reconsidered for re-identification risk. In an embodiment, patient nodes similar to linked nodes are reconsidered. Similar nodes may be found as a side product of the entity resolution. The entity resolution may conclude that two nodes are likely not the same, yet still similar.

Re-calculating a re-identification risk may involve input from a user, e.g., a deidentification expert. The input may comprise quasi identifiers identified for the original datasets and stored, e.g., in metadata or in the knowledge graph. The input may also comprise new information received from the user. The input may comprise an identification of which nodes are quasi identifiers and/or which nodes are sensitive.

The re-identification risk may be computed from all the quasi identifiers from the different datasets. The recomputed re-identification risk may be compared with a risk threshold value. This may be a new risk threshold value, e.g., set by the user. The risk threshold value may be an average of risk threshold values of the original data sets.

Various algorithms are known in the art to compute re-identification risk. For example, a re-identification risk function may be applied to a patient node. The re-identification risk function may comprise a k-anonymity algorithm. The re-identification risk may be computed for one or more selected quasi-identifiers and/or one or more sensitive attributes. Other privacy models that may be used are the so-called l-diversity, and t-closeness.

In an embodiment, re-identification risk is computed from a re-identification risk algorithm, which may be a conventional algorithm, applied to linked nodes as if they were joined. The resulting re-identification risk is then weighed with the likelihood that the linked nodes indeed correspond to the same person. For example, the re-identification risk algorithm may be multiplied by the likelihood that the nodes represent the same entity, e.g., same person.

If a patient node is found that has a re-identification risk that is exceeding a threshold, the knowledge graph may be modified to decrease the re-identification risk for that patient node. For example, modifying the graph may comprise removing edges between a patient node and an attribute. For example, the dashed edge 231 in graph 206 may be removed to decrease information available for the nodes connected to it. For example, edges between a node of the set and an attribute may be added. This may make nodes more similar and thus increase privacy. Care should be taken with this option that the added nodes are not medically too relevant, though. For example, a node may be removed from the knowledge graph. This may be a patient node or an attribute node. For example, labels or tags associated with nodes and/or edges may be removed or altered.

In an embodiment, multiple anonymized patients having re-identification risk exceeding the re-identification risk threshold are collected before joint de-identification. Performing deidentification for multiple nodes together may create a better end result. For example, it may turn out that one particular attribute has a high contribution to the re-identification risk, by removing this attribute the risk for many nodes may be decreased. If the nodes were de-identified one by one a different action may be taken for the different nodes. The end result in that case would be a graph that is more modified than needed.

The system may generate a warning when high re-identification risk is found, e.g., notify a user, or a privacy officer.

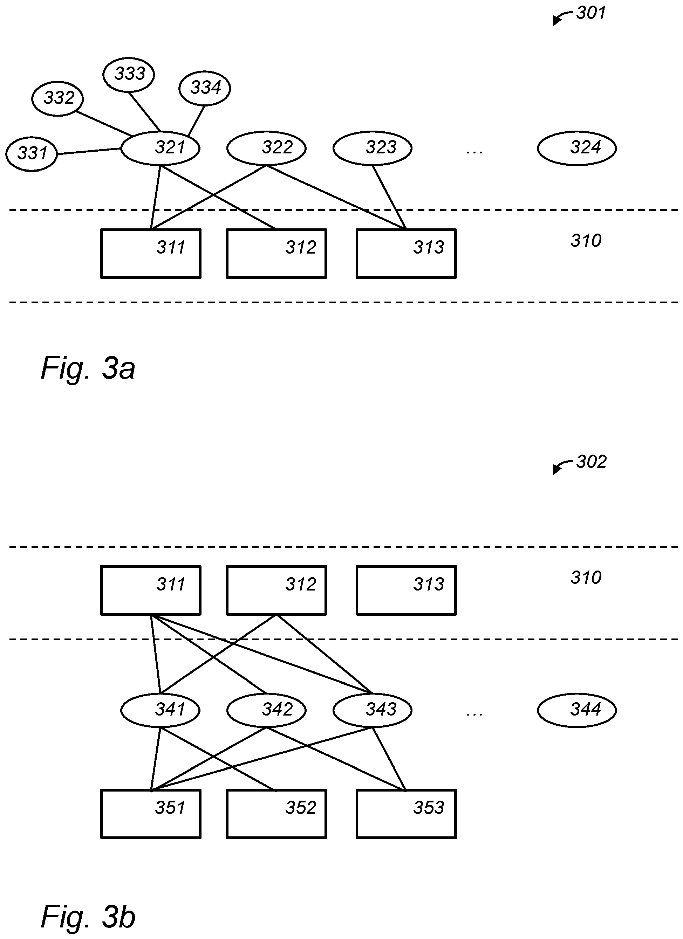

Fig. 3a schematically shows an example of an embodiment of a knowledge graph 301. Knowledge graphs may be generated from a de-identified dataset and linking different entities. As an example, consider the following overview of de-identified data used for building a knowledge graph 301.

| PatientId | Patient Name | Age | Gender | Disease | Symptoms | Heart Rate | Country | Reidentification Risk score |
|---|---|---|---|---|---|---|---|---|
| 1001 | Patient 1 | 91 | M | Stroke, Trauma | Chest Pain | 120 | USA | 0.10 |
| 1002 | Patient 2 | 65 | M | Stroke, Cancer | Chest Pain | 135 | IN | 0.06 |
| 1003 | Patient 3 | 35 | F | Cancer | Fatigue | 105 | UK | 0.08 |

Shown in Fig. 3a are attribution nodes 331-334, patient nodes 321-324, and common attribution nodes 311-313. The patient nodes each correspond to a unique though anonymized patient. For example, patient nodes 321-323 may correspond to patientIds 1001, 1002 and 1003. Attribution nodes 331-334 may correspond to gender, e.g., male, heart rate, e.g., >110, age range, e.g., >90, country, e.g., USA. Attribution nodes 311-313 may correspond to diseases, e.g., stroke, trauma, and cancer respectively. Interestingly, the latter attributes are common, in the sense that they are expected to be present in a further data set as well. For example, these nodes may adhere to a data standard. Instead, common nodes may also be identified manually or with a suitable entity resolution algorithm.

Fig. 3b schematically shows an example of an embodiment of a knowledge graph 302. The knowledge graph 302 is built form a second de-identified data set. As an example, the following data is used. This data set was focused on the diagnosis of memory disorders.

| PatientId | Patient Name | Age | Gender | Disease | Diagnosis | Heart Rate | Reidentification Risk score |
|---|---|---|---|---|---|---|---|
| A9001 | Patient a | 90 | M | Stroke, Trauma | Memory Disorder, Alzheimer's | 120 | 0.10 |
| A9002 | Patient b | 65 | M | Stroke | Memory disorder, dementia | 135 | 0.21 |
| A9004 | Patient c | 35 | F | Stroke, Trauma | Memory disorder, dementia | 105 | 0.11 |

Note that as this dataset is de-identified there is no indication as yet that any of these patients may be the same as any of the patients in the previous data set. Fig. 3b shows the knowledge graph built from only the above data. Knowledge graph 302 shows common attribution nodes 311-313. These are the only nodes that are known to correspond to the same entity as the corresponding nodes in graph 301. Knowledge graph 302 shows patient nodes 341-344 and attribution nodes 351-353.

For example, patient nodes 341-343 may correspond to patient a, b, and c, respectively. Attribution nodes 351-353 may correspond to memory disorder, Alzheimer's, and dementia, respectively.

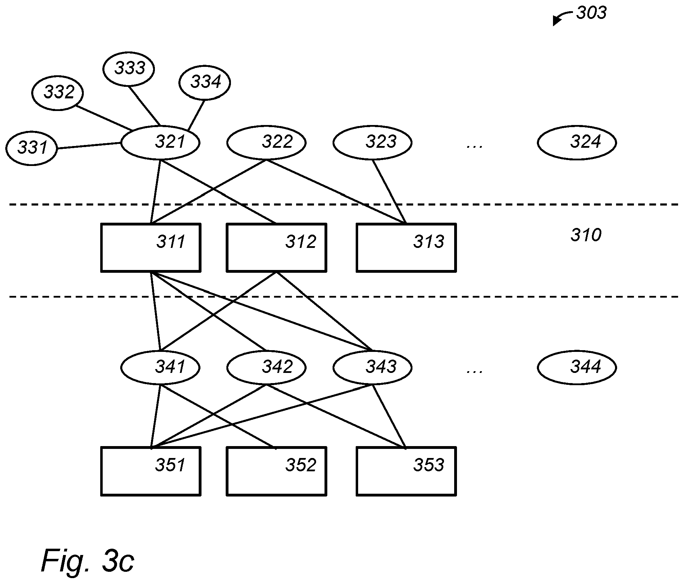

Fig. 3c schematically shows an example of an embodiment of a knowledge graph 303. In knowledge graph 303, the graphs 301 and 302 are merged. In this case common attribution nodes are joined. In this case, the common attribution nodes are given, e.g., an input, e.g., part of metadata. The common nodes may also be identified by an entity resolution algorithm, e.g., a first entity resolution algorithm.

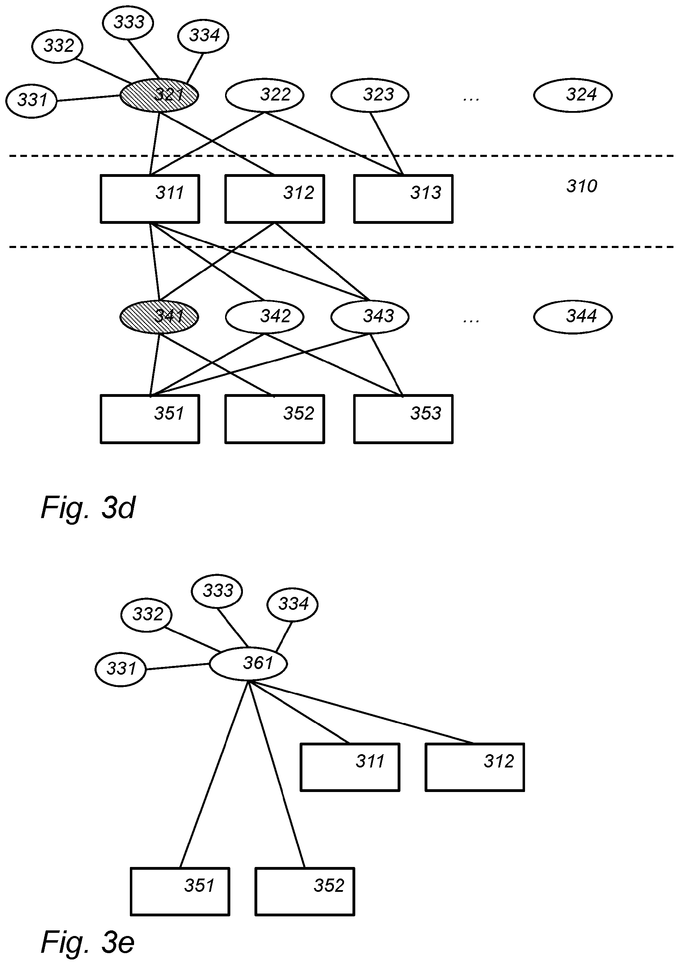

An entity resolution algorithm is now run on the merged graph. The entity resolution algorithm identifies that patient nodes 321 and 341 likely correspond to the same person, this is indicated in Fig. 3d. For example, this determination may be based on a similarity between connection to common nodes. For example, this determination may be based on the fact that both node 321 and node 341 connect to attribute node 311 and node 312. The determination may be further based on the fact that neither attribute node 311 nor node 312 connect to node 313.

Fig. 3e schematically shows a detail of the knowledge graph obtained by joining nodes 321 and 341. Node 361 represents the same patient as node 321 and node 341. Note that the amount of information available for node 361 is higher than that of node 321 and 341. A re-identification risk algorithm may now be run on node 361. If the re-identification risk is too high, de-identification actions may be taken.

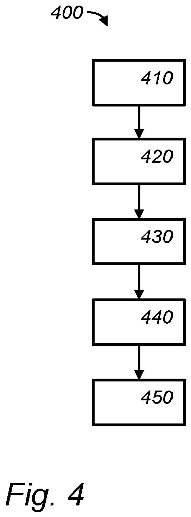

Fig. 4 schematically shows an example of an embodiment of a method 400 for merging multiple de-identified medical datasets. Method 400 is computer-implemented. Method 400 comprises
- obtaining (410) multiple de-identified medical datasets,
- constructing (420) a knowledge graph representing information in the multiple datasets, the knowledge graph having nodes and edges, part of the nodes representing anonymized patients, part of the nodes representing patient attributes, edges representing relationships between the nodes,
- executing (430) an entity resolution algorithm to determine a set of nodes each representing anonymized patients, wherein the anonymized patients corresponding to the nodes in the set correspond to a single anonymized patient with a likelihood exceeding a threshold,
- computing (440) a re-identification risk for the single anonymized patient using the combined attributes connected to the nodes in the set,
- executing (450) a further de-identification for the single anonymized patient to decrease the re-identification risk, conditional upon the re-identification risk exceeding a re-identification risk threshold.

Many different ways of executing the method are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform method 400. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

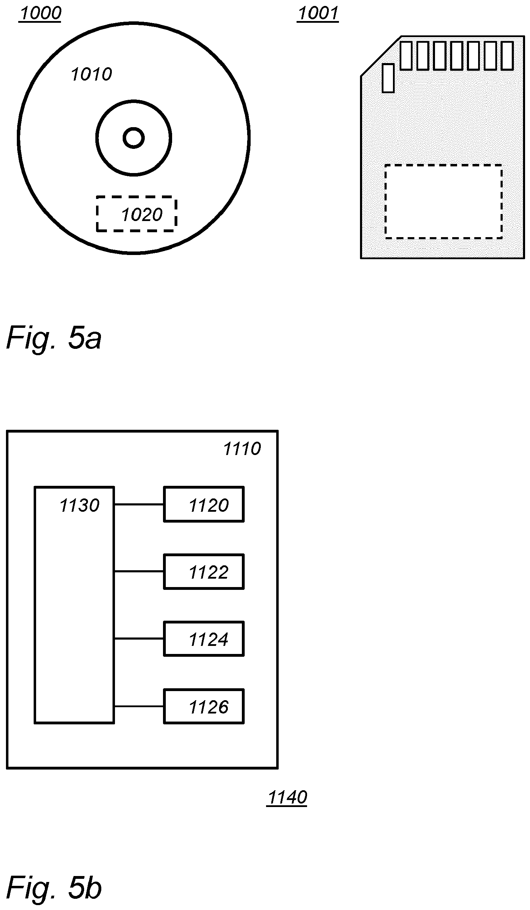

Fig. 5a shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform an embodiment of a method of merging multiple de-identified medical datasets. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform an embodiment of said method of merging multiple de-identified medical datasets.

Fig. 5b shows in a schematic representation of a processor system 1140 according to an embodiment of a system for merging multiple de-identified medical datasets. The processor system comprises one or more integrated circuits 1110. The architecture of the one or more integrated circuits 1110 is schematically shown in Fig. 5b. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the merging device may comprise a processor circuit and a memory circuit, the processor being arranged to execute software stored in the memory circuit. For example, the processor circuit may be an Intel Core i7 processor, ARM Cortex-R8, etc. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

While system 1140 is shown as including one of each described component, the various components may be duplicated in various embodiments. For example, the processing unit 1120 may include multiple microprocessors that are configured to independently execute the methods described herein or are configured to perform steps or subroutines of the methods described herein such that the multiple processors cooperate to achieve the functionality described herein. Further, where the system 1140 is implemented in a cloud computing system, the various hardware components may belong to separate physical systems. For example, the processor 1120 may include a first processor in a first server and a second processor in a second server.

It should be noted that the above-mentioned embodiments illustrate rather than limit the presently disclosed subject matter, and that those skilled in the art will be able to design many alternative embodiments.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of' when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A computer-implemented method (400) for merging multiple de-identified medical datasets, comprising
- obtaining (410) multiple de-identified medical datasets,
- constructing (420) a knowledge graph representing information in the multiple datasets, the knowledge graph having nodes and edges, part of the nodes representing anonymized patients, part of the nodes representing patient attributes, edges representing relationships between the nodes,
- executing (430) an entity resolution algorithm to determine a set of nodes each representing anonymized patients, wherein the anonymized patients corresponding to the nodes in the set correspond to a single anonymized patient with a likelihood exceeding a threshold,
- computing (440) a re-identification risk for the single anonymized patient using the combined attributes connected to the nodes in the set,
- executing (450) a further de-identification for the single anonymized patient to decrease the re-identification risk, conditional upon the re-identification risk exceeding a re-identification risk threshold.

2. A method as in Claim 1, comprising
- receiving one or more de-identified medical datasets, and building an initial knowledge graph,
- constructing the knowledge graph comprises inserting the information in the multiple datasets into the initial knowledge graph.

3. A method as in any of the preceding claims, wherein the entity resolution algorithm links nodes in the set into a single anonymized patient for which re-identification risk is computed.

4. A method as in any of the preceding claims, wherein the further de-identification comprises one or more of
- removing edges between a node of the set and an attribute,
- replacing edges between a node of the set and an attribute,
- removing a node representing an attributed from the knowledge graph,
- removing or altering tags associated with nodes and/or edges.

5. A method as in any of the preceding claims, wherein multiple anonymized patients having re-identification risk exceeding the re-identification risk threshold are collected before joint deidentification.

6. A method as in any of the preceding claims, wherein metadata of the medical datasets is stored, said metadata indicating at least identifiers and quasi-identifiers.

7. A method as in any of the preceding claims, comprising
- computing an initial re-identification risk for anonymized patients in the knowledge graph before executing the entity resolution algorithm,
- collecting anonymized patients having an initial re-identification risk exceeding a threshold, the entity resolution algorithm being executed for the collected patients.

8. A method as in any of the preceding claims, wherein the re-identification risk is computed using the k-anonymity algorithm.

9. A method as in any of the preceding claims, wherein the re-identification risk is computed for one or more selected quasi-identifiers.

10. A method as in any of the preceding claims, wherein
- the set of nodes corresponding to a single anonymized patient with a likelihood exceeding a threshold are joined into a single node, or
- the set of nodes corresponding to a single anonymized patient with a likelihood exceeding a threshold are associated with each other, or
- patient nodes corresponding to a single anonymized patient with a likelihood exceeding a threshold are associated with each other, but attributed nodes corresponding to a single with a likelihood exceeding a threshold are joined into a single node.

11. A method as in any of the preceding claim, wherein a first entity resolution algorithm is performed on attribute nodes, and a second entity resolution algorithm is then performed on patient nodes.

12. A system comprising: one or more processors; and one or more storage devices storing instructions that, when executed by the one or more processors, cause the one or more processors to perform operations for a method according to any of the preceding claims.

13. A non-transitory computer storage medium encoded with instructions that, when executed by one or more computers, cause the one or more computers to perform operations according to any of the preceding claims.
